# EUROPEAN PATENT SPECIFICATION

(11) **EP 1 633 883 B1**
(45) Date of publication and mention of the grant of the patent: **10.09.2008**
(21) Application number: 04742944.4
(22) Date of filing: 17.06.2004
(51) Int. Cl.: C12Q 1/48

(54) **METHOD FOR ASSAYING LKB1 PHOSPHORYLATION ACTIVITY**
VERFAHREN ZUM TESTEN DER LKB1-PHOSPHORYLIERUNGSAKTIVITÄT
PROCEDE DE DOSAGE DE L'ACTIVITE DE PHOSPHORYLATION LKB1

(30) Priority: 17.06.2003 US 479100 P; 23.12.2003 GB 0329866
(43) Date of publication of application: 15.03.2006
(73) Proprietor: Medical Research Council, 20 Park Crescent London W1B 1AL (GB); Columbia University, New York, NY 10032 (US)
(72) Inventor: CARLING, David MRC Clinical Sciences Centre, London W12 0NN (GB); WOODS, Angela MRC Clinical Sciences Centre, London W12 0NN (GB); LEIPER, Fiona MRC Clinical Sciences Centre, London W12 0NN (GB); CARLSON, Marian Columbia University, New York, NY 10032 (US); HONG, Seung, Pyo Columbia University, New York, NY 10032 (US)
(74) Representative: Richards, William John
(86) International application number: PCT/GB2004/002584
(87) International publication number: WO 2004/113562

(56) References cited:
- EP-A- 1 036 844
- WO-A-01/93874
- BAAS A F ET AL: "Activation of the tumour suppressor kinase LKB1 by the STE20-like pseudokinase STRAD." EMBO (EUROPEAN MOLECULAR BIOLOGY ORGANIZATION) JOURNAL, vol. 22, no. 12, 16 June 2003 (2003-06-16), pages 3062-3072, XP002298130 ISSN: 0261-4189
- HARDIE D G ET AL: "AMP-ACTIVATED PROTEIN KINASE: THE ENERGY CHARGE HYPOTHESIS REVISITED" BIOESSAYS, CAMBRIDGE, GB, vol. 23, no. 12, December 2001 (2001-12), pages 1112-1119, XP009024826 ISSN: 0265-9247
- HAWLEY SIMON A ET AL: "Complexes between the LKB1 tumor suppressor, STRADalpha/beta and MO25alpha/beta are upstream kinases in the AMP-activated protein kinase cascade." JOURNAL OF BIOLOGY (ONLINE) 2003, vol. 2, no. 4, 2003, pages 28.1-28.16, XP002298131 ISSN: 1475-4924
- WOODS ANGELA ET AL: "LKB1 is the upstream kinase in the AMP-activated protein kinase cascade." CURRENT BIOLOGY, vol. 13, no. 22, 11 November 2003 (2003-11-11), pages 2004-2008, XP002298132 ISSN: 0960-9822
- CARLING D: "The AMP-activated protein kinase cascade - a unifying system for energy control" TIBS TRENDS IN BIOCHEMICAL SCIENCES, ELSEVIER PUBLICATION, CAMBRIDGE, EN, vol. 29, no. 1, January 2004 (2004-01), pages 18-24, XP004483822 ISSN: 0968-0004
- LIZCANO JOSE M ET AL: "LKB1 is a master kinase that activates 13 kinases of the AMPK subfamily, including MARK/PAR-1." THE EMBO JOURNAL. 25 FEB 2004, vol. 23, no. 4, 25 February 2004 (2004-02-25), pages 833-843, XP002298133 ISSN: 0261-4189
- SHAW REUBEN J ET AL: "The tumor suppressor LKB1 kinase directly activates AMP-activated kinase and regulates apoptosis in response to energy stress." PROCEEDINGS OF THE NATIONAL ACADEMY OF SCIENCES OF THE UNITED STATES OF AMERICA, vol. 101, no. 10, 9 March 2004 (2004-03-09), pages 3329-3335, XP002298134 ISSN: 0027-8424
- BOUDEAU JEROME ET AL: "Heat-shock protein 90 and Cdc37 interact with LKB1 and regulate its stability." BIOCHEMICAL JOURNAL, vol. 370, no. 3, 15 March 2003 (2003-03-15), pages 849-857, XP002298135 ISSN: 0264-6021

## Description

### Background To The Invention

Peutz-Jeghers Syndrome (PJS) is a hereditary cancer syndrome which is characterised by a predisposition to both benign and malignant tumours of many organ systems. This syndrome is though to be linked to loss-of-function mutations in protein kinase LKB1 (GenBank accession number U63333 - see Hemminki et al. 1998 Nature vol 391 pp185-187).

No satisfactory assay exists for the inter-molecular kinase activity of LKB1 in the prior art.

Prior art assays for LKB1 activity include autophosphorylation assays such as those presented by Boudeau et al. 2003 Human Mutation:Mutation in Brief 583. Regardless of the physiological significance of the autophosphorylation on Thr336 of LKB1, which is at best unclear, no physiological substrates of LKB1 are known in the prior art (eg. see Boudeau page 8 and elsewhere), let alone any kinase substrate of LKB 1.

Moreover, prior art attempts to study LKB1 activity focus on loss-of-function mutants such as gross deletions of catalytic part(s) of the molecule. So little is known about LKB1 in the prior art, that such crude experiments are the best available route of investigation. Progress is hampered by lack of connection of LKB1 with downstream components of the signalling/metabolic pathways.

Even if autophosphorylation of LKB1 is a significant biological event, ablating this activity and studying the negative effects is a crude instrument of investigation and cannot lead to the analysis of downstream components without meaningful and biologically significant substrates for LKB1.

Since no significant LKB1 substrates are known in the prior art, it is impossible to design a robust assay for LKB1 activity or activation. It is also not possible to dissect the significance of parts of LKB1 itself, such as the autophosphorylation event, which may be merely permissive rather than regulatory.

Investigation of Peutz-Jeghers Syndrome (PJS) is limited by what is known about the underlying genetic basis of the disease. The prior art discusses links to LKB1 (eg. see Hemminki et al. 1998 Nature vol 391 pp185-187). However, links to downstream targets or effectors have not been established in the prior art. Furthermore, downstream targets or effectors of LKB1 have not been established in the prior art. Thus, there is a lack of suitable targets for intervention such as in the treatment or prevention of PJS.

AMPK activation is known to be correlated with its phosphorylation state (eg. see Neumann et al. 2003 Protein Expression and Purification 'Mammalian AMP-activated protein kinase: functional, heterotrimeric complexes by co-expression of subunits in *E.coli'*). However, a kinase responsible for this phosphorylation has not been identified.

The prior art has attempted to provide techniques for the activation of AMPK, but in the absence of a known activator of AMPK, these techniques are confined to the partial purification of cell extracts which are then applied to AMPK in vitro with the hope that said extracts comprise some active form of an unknown upstream kinase which may result in AMPK activation. These experiments, whilst representing the state of the art, are nevertheless labour intensive and necessarily crude in nature, being based on incompletely characterised cellular extracts. Furthermore, these experiments involve sacrifice of animals which, although minimised, is nevertheless undesirable.

Baas et al (2003 EMBO vol 22 pp3062-3072) disclose activation of the tumour suppressor kinase LKB1 by the STE20-like pseudokinase STRAD.

EP1036844A1 discloses an examination method, examination reagent and remedy for diseases caused by variation in LKB1 gene.

WO01/93874 discloses a method of treatment of obesity and paralysed muscle and ergogenic aids.

Hardie and Hawley (2001 Bioessays vol 23 pages 1112-1119) disclose AMP-activated protein kinase and its involvement in the energy charge hypothesis.

The present invention seeks to overcome problem(s) associated with the prior art.

### Summary Of The Invention

The present invention is based on the surprising finding that LKB phosphorylates downstream kinase(s). Furthermore, LKB1 is in the same cognate pathway as AMPK.

More in particular, the invention is founded on the surprising finding that LKB1 acts on AMPK. Furthermore, as is demonstrated herein, LKB1 causes phosphorylation and activation of AMPK. As explained below, LKB1 can exert its action by direct phosphorylation of AMPK and it is shown for the first time that LKB1 has a specific cellular kinase substrate (eg. AMPK), and conversely that AMPK is targeted and activated by the upstream kinase LKB 1.

These surprising findings have allowed the provision of numerous assays, compositions and treatments which are detailed below and in the appended claims.

Thus, in one aspect, the invention provides an *in vitro* method for assaying LKB1 activity comprising
(i) providing a sample comprising LKB1,
(ii) contacting said sample with a substrate kinase under conditions permissive of phosphorylation, wherein said substrate kinase is or is derived from AMPK, and
(iii) monitoring incorporation of phosphate into said substrate kinase
wherein the incorporation of phosphate into said substrate kinase indicates LKB1 activity.

We show that AMPK is a particularly good substrate kinase for LKB1. Thus, in another aspect, the invention relates to a method as described above wherein said substrate kinase is or is derived from AMPK.

AMPK is considered to be a heterotrimeric complex in vivo. It is therefore advantageous to use heterotrimeric AMPK when assaying LKB1. Thus, in another aspect, the invention relates to a method as described above wherein the AMPK comprises heterotrimeric AMPK. Preferably the AMPK comprises mouse or human AMPK, preferably human AMPK.

Phosphorylation of AMPK may be assayed by any suitable method such as assay of AMPK activation. In another aspect, the invention relates to a method as described above wherein incorporation of phosphate into AMPK is monitored by assaying AMPK activity. Preferably AMPK activity is assayed via phosphorylation of SAMS peptide.

Phosphorylation of AMPK may also be assayed by direct readout of AMPK phosphorylation state. This may be done using any suitable method such as radio labelled phosphate, or by using specific reagents to assay the phosphorylation state of AMPK directly. Preferably specific reagents are used. Thus, in another aspect, the invention relates to a method as described above wherein incorporation of phosphate into AMPK is monitored by assaying phosphorylation of T-172 of AMPK, or the equivalent thereof. The equivalent means the same residue in an isoform or homologue of AMPK, or may include a different residue (eg. serine) at the same postion in AMPK. Preferably equivalent means functionally equivalent ie. indicative of AMPK activation.

Advantageously the phosphate incorporation into AMPK may be quantified to give an estimate of LKB1 activity. Techniques for quantitative kinase assay are well known in the art. Phosphate incorporation may be quantified by phosphorimaging or by densitometry, or by similar techniques applied to direct immunological assay of T-172. Thus, in another aspect, the invention relates to a method as described above further comprising quantifying the amount of phosphate incorporated per mol of AMPK, wherein said phosphate level indicates the level of LKB1 present in said sample.

It is surprisingly disclosed herein that LKB1 can be used to phosphorylate and activate AMPK. Thus, in another aspect, the invention relates to *in vitro* use of recombinant or isolated LKB1 in the activation of AMPK.

In another aspect, the invention relates to *in vitro* use of recombinant or isolated LKB1 as an AMPKK.

In another aspect, the invention relates to *in vitro* use of recombinant or isolated LKB1 in the phosphorylation of AMPK.
In another aspect, the invention relates to use of LKB1 in the manufacture of a medicament for the prevention or treatment of Peutz-Jeghers Syndrome (PJS).

It is surprisingly disclosed herein that AMPK lies in the same cognate pathway as LKB1, and is directly activated by action of LKB 1.

It is disclosed herein that LKB1 increases the phosphorylation of AMPK.

In another aspect, the invention relates to use of AMPK in the manufacture of a medicament for the prevention or treatment of hypercardiomyotrophy (HCM).

There are certain molecules known to stabilise and/or activate LKB 1. It is therefore advantageous to use these molecule(s) in the activation and/or maintenance of AMPK. Thus the present invention relates to *in vitro* use of modulators of LKB1 in the modulation of AMPK. In another aspect, the invention relates to an *in vitro* method of modulating AMPK comprising modulating LKB1.

In another aspect, the invention relates to *in vitro* use of HSP90 in the modulation of AMPK activity.

In another aspect, the invention relates to *in vitro* use of Cdc37 in the modulation of AMPK activity.

In another aspect, the invention relates to *in vitro* use of STRAD protein in the modulation of AMPK activity.

In another aspect, the invention relates to use of LKB1 in the manufacture of a medicament for the prevention or treatment of Peutz-Jeghers Syndrome (PJS).

In another aspect, the invention relates to an *in vitro* method for phosphorylating AMPK comprising contacting AMPK with recombinant or isolated LKB 1.

In another aspect, the invention relates to an *in vitro* method of increasing the phosphorylation of AMPK in a system comprising increasing the activity of LKB1 in said system.

In another aspect, the invention relates to an *in vitro* method of activating AMPK in a system comprising activating LKB1 in said system.

In another aspect, the invention relates to an *in vitro* method of activating AMPK comprising contacting said AMPK with recombinant or isolated LKB1.

In another aspect, the invention relates to an *in vitro* method of phosphorylating AMPK comprising contacting said AMPK with recombinant or isolated LKB 1.

In another aspect, the invention relates to an in vitro method for identification of modulator(s) of LKB1 comprising providing a first and a second sample of LKB1, contacting said first sample with a candidate modulator of LKB1, contacting said first and second samples with a substrate under conditions permissive of phosphorylation, monitoring incorporation of phosphate into said substrate, and comparing the incorporation of phosphate into said substrate in said first and second samples, wherein if the incorporation of phosphate into the substrate differs between said first and second
samples, the candidate modulator is identified as a modulator of LKB1 activity. Preferably the substrate is a substrate kinase. Preferably the substrate kinase is or is derived from AMPK. In another aspect, the invention relates to modulators of LKB1 identified by this method. Preferably said modulators are activators.

In another aspect, the invention relates to recombinant eukaryotes for the study of AMPK/AMPKK. For example, the invention advantageously provides yeast cells distrupted for AMPKKs. Thus, In another aspect, the invention relates to a recombinant yeast cell comprising gene disruptions in at least two of Pak1, Tos3 and Elm1. Preferably the recombinant yeast cell comprising gene disruptions in Pak1, Tos3 and Elml. Preferably the recombinant yeast cell further comprises a gene disruption in Snf1. Preferably the recombinant yeast cell further comprises a gene disruption in Snf4. Preferably the recombinant yeast cell further comprises a gene disruption in SIP1, SIP2 and GAL83. Preferably the recombinant yeast cell is capable of expressing mammalian AMPK. Preferably the recombinant yeast cell expresses mammalian AMPK. Preferably the mammalian AMPK is mouse or human AMPK, preferably human AMPK. Preferably the mammalian AMPK comprises alpha, beta and gamma subunits. Preferably the yeast is *Saccharomyces cerevisiae.* The gene disruptions may be made by any suitable technique, or may be assembled together into the same cell from individual strains by genetic crossing.

In another aspect, the invention relates to complementation cloning of AMPKKs and/or AMPKs by use of these strains, eg. by transformation with a candidate AMPK/AMPKK or library thereof and selection on carbon sources to isolate functional AMPKs and/or AMPKKs. In another aspect, the invention relates to AMPKs/AMPKKs isolated in this manner.

### Detailed Description of the Invention

We have identified a protein kinase that phosphorylates and activates AMP-activated protein kinase (AMPK). This forms a therapeutic target for compounds aimed at modulating AMPK, in particular activating AMPK.

The invention finds particular utility in the treatment or prevention of one or more of type 2 diabetes, Peutz-Jeghers syndrome, cancer and obesity. Further applications and details are provided below.

Identification of AMPK as a downstream substrate of LKB1 enables provision of an assay system for screening compounds that modulate and/or activate the upstream kinase and which may have a therapeutic role in the treatment of cancer or other disorders as explained herein.

Identification of an upstream kinase in the AMPK cascade provides a target for candidate drug screens aimed at treating type 2 diabetes and obesity, as well as other disorders described herein.

Identification of AMPK as a substrate for LKB1 further provides an assay for screening candidate drugs that activate LKB1. This has application in cancer therapy as well as metabolic diseases and other disorders.

In broad aspect, the invention relates to a method for assaying LKB1 activity comprising providing a sample of LKB1, contacting said sample with a substrate under conditions permissive of phosphorylation and monitoring incorporation of phosphate into said substrate, wherein the incorporation of phosphate into said substrate indicates LKB1 activity. In this aspect, the substrate means any biologically significant substrate such as one having a demonstrable biological effect such as those demonstrated herein (see Examples section). Preferably said substrate is itself a kinase (ie. a 'substrate kinase'), preferably said substrate is or is derived from AMPK, preferably said substrate is AMPK.

As used herein the term 'substrate kinase' has its ordinary meaning. In particular, the 'kinase' means a recognisable kinase by sequence comparison, such as possessing kinase signature motif(s) as defined by Hunter (eg. see Hanks, Quinn and Hunter 1988 Science vol 241 p.42). Naturally, 'kinase' does not imply exlcusion of kinase dead variants or catalytically inactive fragment(s), but is used to describe that family of proteins which are ordinarily classified as kinases with reference to Hanks et al. Preferably kinase means protein and/or sugar and/or lipid kinase, preferably protein kinase.

As used herein, 'system' has its natural meaning, and may include complex biological system(s) such as whole cell(s).

The term 'derived from' has its normal meaning in the art, wherein a substance is considered to be 'derived from' a first substance when part of the substance has been created or constructed through a chain of events which incorporates all or part of the first substance into the substance in question. Naturally the two substances are likely to differ eg. through mutation, addition or deletion or similar modification, but if the substance in question has inherited features from the first substance then it is derived from it. In particular, when used in connection with biopolymers such as polynucleotide(s) or polypeptide(s), a substance is considered to be derived from a first substance when it possesses sufficient sequence identity to be recognised as related to the first substance. In this context, if a substance is derived from a first substance, then said substance preferably has at least 10 contiguous residues which possess at least 25% identity with the first substance, preferably 30% identity, preferably 40% identity, preferably 50% identity, preferably 60% identity, preferably 70% identity, preferably 80% identity, preferably 90% identity, preferably 95% identity, preferably 96% identity, preferably 97% identity, preferably 98% identity, preferably 99% identity or even more. Preferably said substance has at least 15 contiguous residues with said identity, preferably at least 20 residues, preferably at least 30 residues, preferably at least 50 residues, preferably at least 100 residues, preferably at least 200 residues, or even more. For multimeric entities, the term may be applied to the complex and/or to individual components as will be apparent from the context. Generally it will be enough if one of the subunits is derived from the given entity.

A homologue is as usually understood in the art. Preferably homologue refers to the equivalent from another organism such as another species of organism.

The term 'isolated' may mean recombinant and/or purified. When purified, the term refers to the substance when separated from at least one component of the composition in which it is naturally found.

The agent/modulator of the present invention refers to entities involved in the activation of and/or phosphorylation of AMPK. An exemplary agent/modulator of the present invention is LKB1. This may be used in combination with stabilising agent(s) such as HSP90 and/or Cdc37, or combinations thereof. Another modulator is STRAD protein which can activate LKB1 and therefore activate AMPK. Thus the invention relates to the use of STRAD protein in the modulation of AMPK, preferably in the activation of AMPK.

### Assaying AMPK activity

There are a variety of ways in which AMPK may be assayed by a person skilled in the art. In accordance with the present invention, any suitable technique for assay of AMPK activity may be employed.

AMPK activity may be determined directly (eg. by assaying kinase activity of AMPK) or indirectly (eg. by assaying the phosphorylation state of threonine 172 of AMPK). Preferably AMPK activity is measured directly.

The majority of techniques for assaying AMPK rely on measuring phosphorylation of a substrate capable of being phosphorylated by AMPK. This substrate may be any suitable substrate such as a polypeptide (eg. a relatively short substrate peptide (eg. 50 amino acid residues or less) or a longer polypeptide/protein or fragment or domain thereof.

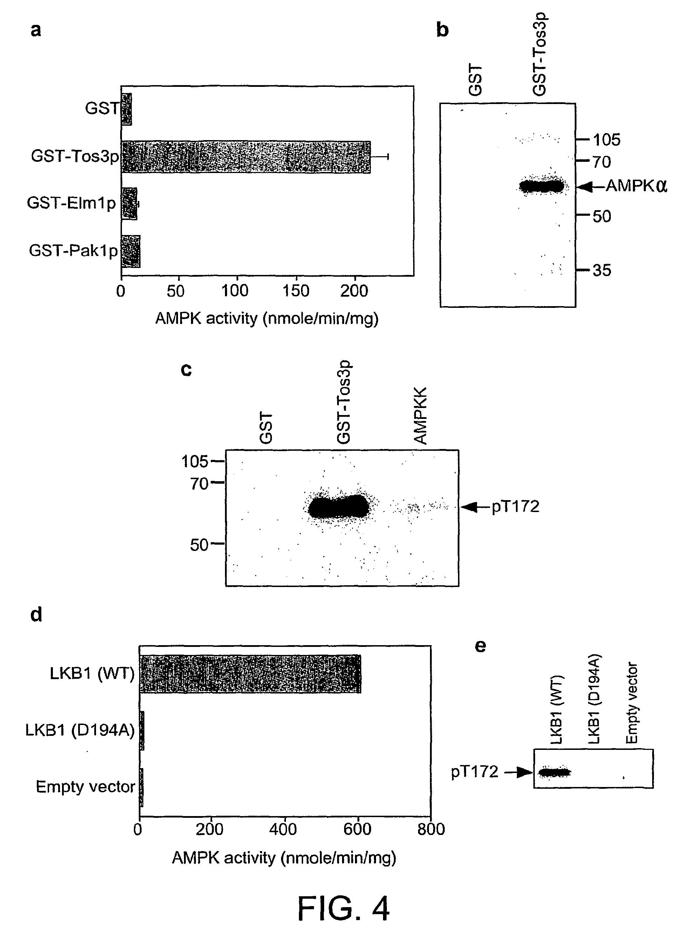

AMPK activity can be assayed by measuring the transfer of phosphate to an AMPK substrate by AMPK. It is also possible to assay AMPK activity by determining the phosphorylation state of residue 172 (preferably the wild-type threonine 172) within the alpha subunit of AMPK. Examples of this assay are given below, eg. as shown in Fig. 4e.

Using this method AMPK is phosphorylated by the upstream kinase and phosphorylation of threonine 172 determined by Western blotting using an antibody that specifically recognises phospho-threonine 172 (available commercially from Cell Signalling Technologies).

AMPK activity is preferably assayed by measuring phosphorylation of a substrate (e.g. a peptide or a protein substrate). This procedure is a very standard one for protein kinases. AMPK can be assayed using protein substrates (e.g. acetyl-CoA carboxylase) as well as shorter peptides.

The transfer of phosphate is preferably monitored using radioisotope(s) of phosphorus. For example, 32-P labelled ATP may be used, but it could be any labelled phosphate e.g. 33-P. Preferably 32-P gamma labelled ATP is used.

The AMPK substrate is preferably a peptide. This peptide may be any peptide substrate that is capable of being phosphorylated by AMPK.

Preferably the peptide comprises a consensus sequence for phosphorylation by AMPK, said consensus comprising a hydrophobic residue 5 amino acids N-terminal to a phosphorylatable residue (which is either a serine or a threonine), a basic residue at a position either 2, 3 or 4 amino acids N-terminal to the phosphorylatable residue with a hydrophobic residue 4 amino acids C-terminal to the phosphorylatable residue, which maybe represented as:
hydrophobic-(basic,X, X)-X-serine or threonine-X-X-X-hydrophobic
where the order of the amino acids in brackets is not critical.

Further guidance on the consensus motif for phosphorylation by AMPK may be found in Weekes, J., Ball, K.L., Caudwell, F.B. and Hardie, D.G. FEBS Lett. 334, 335-339 (1993), which is incorporated herein by reference.

These peptides may be readily synthesised by any suitable technique, or may be sourced from commercial companies or service laboratories, or be simply made according to need on an ad hoc basis.

A preferred example of a suitable AMPK substrate is the SAMS peptide (HMRSAMSGLHLVKRR) (Davies, S.P., Carling, D. and Hardie, D.G. Eur. J. Biochem. 186, 123-128 (1989). This is available commercially from Upstate Biotechnology Inc.

Preferably AMPK activity is assayed as described herein (eg. as shown in Figure 4d).

AMPK may also be assayed by genetic means. For example, an assay for yeast AMPK (Snfl) in which transcription of a *lexAop-lacZ* reporter depends on the catalytic activity of LexA-Snflp bound to the promoter (Kuchin, 2000- Kuchin, S., Treich, I. & Carlson, M. A regulatory shortcut between the Snf1 protein kinase and RNA polymerase II holoenzyme. Proc. Natl. Acad. Sci. USA 97, 7916-7920 (2000) - incorporated herein by reference) may be used or adapted. This and other genetic tests are explained more fully below, such as in the examples section.

### AMPK

With regard to the use of AMPK in the assays of the present invention there are many alternative methods for producing the proteins available to the skilled person.

AMPK, or homologues of AMPK, that contain the phosphorylatable residue necessary for activation, and which contain those amino acid residues necessary for activity, could be used in an assay according to the present invention. Preferably the activation residue is 172, preferably threonine 172. Whether a residue is necessary for activity may be determined by testing in AMPK assay(s) as described herein, for example using the SAMS peptide.

AMPK is activated by phosphorylation in the activation loop. In particular, AMPK is activated by phosphorylation on the activation site 172. Naturally occurring AMPK usually possesses threonine at this site, however it will be appreciated that a similar phosphoacceptor amino acid may be substituted at this site, eg. serine. Furthermore, constitutively active mutants may be constructed by further mutation at this site as is known in the art, eg. introducing negatively charged residues. In the context of the present invention, AMPK preferably possesses a phosphoacceptor residue in the activation loop at position 172 as judged by reference to the wild type mammalian sequence, and preferably position 172 is threonine.

In this regard, any active preparation of LKB1, eg. recombinant LKB1 expressed in a suitable system with or without tag sequence(s), may be used to phosphorylate and activate any form of AMPK which retains the activation site (equivalent to threonine 172) and/or was capable of displaying catalytic activity of AMPK following phosphorylation.

It will be appreciated that AMPK is naturally composed of multiple subunits. Thus, as used herein, the term 'AMPK' refers to that protein or association of proteins which has the AMPK activity. Currently there are at least 12 possible combinations of subunits in association to form AMPK. This number follows from the fact that there are currently 2 alpha subunits, 2 beta subunits and 3 gamma subunits, thereby giving a possible 12 different heterotrimeric forms of AMPK. The alpha subunit is targeted by LKB1.

The preferred source of AMPK useful in the present invention is a bacterially expressed mammalian AMPK complex. A preferred strategy used for expression of the complex is detailed in Neumann et al. 2003 Protein Expression and Purification 'Mammalian AMP-activated protein kinase:functional, heterotrimeric complexes by co-expression of subunits in *E.coli'.* Thus, in a preferred embodiment, the AMPK used in the assays of the present invention is prepared according to Neumann et al.

It will be appreciated that although Neumann et al. works from mouse AMPK subunits encoded by cDNAs detailed in GenBank accession numbers X95578, X95577 and U40819, preparation of human AMPK subunits is well within the ability of the person skilled in the art by following Neumann and simply modifying the PCR primers used for the cloning steps to track the human sequence as necessary, and using a suitable human template nucleic acid for the PCR step. Thus, in another embodiment, preferably the AMPK is human AMPK prepared according to Neumann et al.

It is also possible to use truncated version(s) of the AMPK alpha subunit in the present invention, preferably those which contain the catalytic subunit, preferably including threonine 172. By truncating AMPK alpha it is possible to produce a protein that can be phosphorylated by the upstream kinase on threonine 172 and is active in the absence of the other 2 regulatory AMPK subunits (beta and gamma). Preferably truncated AMPK alpha for use in the present invention is prepared according to Hamilton, S.R., O'Donnell, J.B., Hammet, A., Stapleton, D., Habinowski, S.A., Means, A.R., Kemp, B.E. and Witters, L.A. Biochem. Biophys. Res. Commun. 293, 892-898 (2002). This material comprises the first 312 amino acids of alpha1 bacterially expressed and purified using Ni-NTA. This material may then be used as a substrate for the upstream kinase in the assays of the present invention.

It is also possible to use AMPK purified from animal tissues. For example AMPK may be purified from rat liver according to Carling, D., Clarke, P.R., Zammit, V.A. and Hardie, D.G. Eur. J. Biochem. 186, 123-128 (1989). Naturally it will be appreciated by the skilled reader that it is possible to use in the assays of the present invention AMPK purified from any source containing the enzyme. For example, purified AMPK for use in the present invention is available commercially from Upstate Biotechnology Inc.

Furthermore, it will be appreciated that homologue(s) of AMPK from other species would also be useful in the present invention e.g. SNF1 from yeast.

In a preferred embodiment, AMPK may be prepared for use in the assays of the present invention as described in Neumann *et al* (Neumann, Woods, Carling, Wallimann and Schlattner Protein Expression and Purification 2003 "Mammalian AMP-activated protein kinase: functional, heterotrimeric complexes by co-expression of subunits in *E. coli"*).

In another preferred embodiment, AMPK may be prepared according to Hamilton et al. (Hamilton et al. 2002 BBRC vol 293 pp892-898) which describes expression of a truncated form of AMPK suitable for use in the present invention.

### LKB1

Regarding the use of LKB1 in the assays of the present invention there are many alternative methods for producing LKB1 available to the skilled reader. Naturally the sample may not be known to comprise LKB1, eg. if the assay is being used qualitatively to determine if LKB1 activity is present in said sample.

Any active preparation of LKB1 (eg. expressed in a suitable system with or without any tag sequence(s)) could be used to phosphorylate and activate any AMPK preparation such as AMPK that retains the activation site (equivalent to threonine 172 of AMPK) and/or was capable of exhibiting catalytic activity following phosphorylation (see AMPK section above).

Specifically, Sapkota et al. (Sapkota et al 2001, JBC vol 276 pp19469-19482) provide examples of different tagged forms of LKB 1 suitable for use in the present invention. Preferably the LKB1 is produced in E.coli and purified according to Sapkota et al., which is incorporated herein by reference.

It will be appreciated by the skilled reader that different clones of LKB1 from different species would also be suitable. These may include homologues of LKB 1. Preferred are those homologues exhibiting greatest sequence identity to human or mouse LKB1, preferably to human LKB 1.

Sapkota uses mouse sequence NCBI AA542163/IMAGE 550355 as template in the PCR cloning of LKB1. Naturally, the person skilled in the art will appreciate that Sapkota is easily adapted to provide the human LKB1 by following the same procedures, and simply adapting the primer sequences as necessary to the human sequence, and using a suitable human template nucleic acid. Thus, in one embodiment, LKB is preferably human LKB.

It may be desirable to use a truncated version of LKB1 that retains activity. Retention of LKB activity is easily determined using the assays of the present invention.

LKB may be activated as described below, eg. by expression in particular cell line(s).

It may be desirable to express LKB1 directly in an active form and this could then be used in the assays of the present invention. LKB1 may be activated and/or stabilised as is known in the art, eg using HSP90 and/or Cdc37 and/or STRAD protein.

LKB1 is known to be active when expressed in COS7, HEK293, G361 melanoma, CCL13 liver hepatoma, H2K skeletal muscle, HeLa or Sf9 cells.

In a preferred embodiment, the LKB and the AMPK would be from, or would be derived from, the same species.

### LKB1 Assay

LKB1 kinase assay is conducted using standard conditions for kinase assays. The commentary below refers to final concentrations in the assay.

pH is preferably buffered for LKB1 kinase assays. Preferably the pH is buffered between 6 and 9, preferably between 6.5 and 8.5, preferably 7.5.

The buffering agent may be Tris, HEPES, MOPS, MES or other suitable buffer agent. Preferably the agent is HEPES. Preferably the buffer agent is 50mM.

Metal ion is included. Preferably 2+ metal ion. Preferably manganese and/or magnesium ion. Preferably magnesium ion. Preferably metal ion is provided as metal chloride. Preferably the metal ion is present at 1-10mM, preferably 5mM.

Stabiliser is optionally included. Stabiliser may be glycerol eg. 10% glycerol, or may be a detergent such as triton eg. 1% triton or tween eg. 0.1 % tween.

Reducing agent is advantageously included. Reducing agent may be DTT, glutathione, B-mercaptoethanol or other suitable agent. Preferably the agent is used at 1mM. Preferably the agent is DTT.

Chelating agent is optionally used. Preferably the chelating agent is EDTA, preferably 1mM EDTA.

ATP is included. Preferably ATP is present at 10uM to 10mM, preferably 10uM to 5mM, preferably 100um. ATP may be labelled (preferably gamma labelled), eg. with 33P or 32P, preferably 32P, or may be unlabelled depending upon needs and chosen read-out. If unlabelled, read-out is preferably immunodetection of P-T172 of AMPK.

Activator(s) of LKB 1 may optionally be included. These may include STRAD protein and/or AMP.

Other LKB1 factors may be included if desired eg. HSP90, Cdc37 or others.

Preferably conditions for LKB1 assay are as in the examples section, particularly preferred are those conditions given in Example 7 such as 100uM ATP,

### Gene Disruption

Gene disruption may be accomplished by any technique known to those skilled in the art, such as interruption, mutation or deletion. Preferably gene disruption is accomplished by deletion of at least part of the coding sequence from the genome, preferably by deletion of the entire coding sequence from the genome.

Disruption may also be accomplished by modification of transcriptional elements, such as promoter element(s) or by over-expression of antisense or inhibitory nucleic acid(s) to reduce or abolish translation of a transcribed RNA, or by any other suitable means known in the art.

### Tag sequences

The polypeptides useful in the present invention may be prepared with an epitope tag, such as myc, HA, glutathione-*S*-transferase, flag or other tag. Preferred is flag.

### Medical Applications

The present invention finds particular utility in the treatment, prevention and/or amelioration of Peutz-Jeghers syndrome (PJS), Obesity, diabetes, preferably type II diabetes, Hypercardiomyotrophy (HCM), maintenance of cellular energy homeostasis, stress including heat shock, hypoxia and ischemia, muscular fatigue and other states connected to compromised cellular ATP levels, cancer, particularly cancers occurring in PJS subjects, intestinal polyposis, resistance to transformation and suppression of cell growth eg. reversal/arrest of deregulated growth state(s), induction of or permission of apoptotic death.

Snfl/AMPK family of kinases is important for metabolic stress responses in eukaryotes. In mammals, AMPK is activated by multiple stresses, many of which lead to an increase in the cellular AMP:ATP ratio, and also by leptin and the hypoglycemic agent metformin. AMPK has a central role in co-ordinating energy homeostasis and is a major regulator of lipid metabolism, glycogen storage, and glucose transport. AMPK has been implicated in the development and treatment of metabolic disorders, including type 2 diabetes and obesity, and mutations in AMPK cause cardiac abnormalities in humans.

In particular, the treatments of the present invention may be used in a combined regime with p53 targeted drugs (eg. see Bardeesy et al Nature vol 419 p162) for enhanced effect.

### Activation of yeast Snf1 and mammalian AMP-activated protein kinase by upstream kinases

We have identified three kinases, Tos3p, Pak1p, and Elmlp, that function as upstream kinases in the Snf1 kinase cascade. We present genetic and biochemical evidence that these kinases phosphorylate and activate Snf1 kinase in vitro and in vivo (see examples section). Mutant yeast cells lacking these three kinases are defective in the utilisation of carbon sources other than glucose, a signature function of the Snf1 kinase pathway. This is the first identification of upstream kinases in the Snf1/AMPK kinase cascade with demonstrated physiological roles/functions.

These three kinases clearly exhibit significant functional redundancy, as all three must be mutated to confer the Snf phenotypes examined here. However, it seems likely that they will prove to have some distinct functions with respect to regulation of Snf1. Each of the three upstream kinases may exhibit some preference for Snf1 kinase containing a specific β subunit isoform. Another possibility is that the three kinases respond preferentially to different kinds of metabolic stress or to different signals resulting from a single stress, such as glucose limitation.

As explained herein, the identification of these kinases also has significance beyond yeast, in enabling design and refinement of assays according to the present invention, such as assays and/or screens for the identification of upstream kinases in the AMPK cascade.

Examination of closely related mammalian kinase LKB1 (a tumour suppressor kinase), yielded evidence that LKB1 is an upstream kinase for AMPK.

It is demonstrated herein that LKB1 phosphorylates and activates AMPK on the activation-loop threonine residue in vitro.

LKB1 is thus demonstrated that LKB1 is a valuable target for candidate drugs to influence AMPK activity in cells.

Moreover, these findings support the disclosed role for AMPK in regulation of growth and transformation as well as metabolism, as well as the physiological role of LKB 1 in the regulation of AMPK in vivo.

It will be appreciated that other mammalian kinases which are related to the yeast upstream kinases are also candidates for bona fide kinases of the AMPK cascade according to the present invention. These mammalian upstream kinases represent therapeutic targets according to the present invention, particularly for the treatment of human metabolic disorders, including obesity and type 2 diabetes, and cancers.

### Pharmaceutical Compositions

The present invention also provides a pharmaceutical composition comprising a therapeutically effective amount of the agent(s) and/or modulator(s) of the present invention and a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

Where the agent is to be administered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some embodiments, the agents and/or growth factors of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

If the agent/modulator is a protein, then said protein may be prepared *in situ* in the subject being treated. In this respect, nucleotide sequences encoding said protein may be delivered by use of non-viral techniques (e.g. by use of liposomes) and/or viral techniques (e.g. by use of retroviral vectors) such that the said protein is expressed from said nucleotide sequence.

In a preferred embodiment, the pharmaceutical of the present invention is administered topically.

Hence, preferably the pharmaceutical is in a form that is suitable for topical delivery.

### Administration

The term "administered" includes delivery by viral or non-viral techniques. Viral delivery mechanisms include but are not limited to adenoviral vectors, adeno-associated viral (AAV) vectors, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof.

The components of the present invention may be administered alone but will generally be administered as a pharmaceutical composition - e.g. when the components are is in admixture with a suitable pharmaceutical excipient, diluent or carrier selected with regard to the intended route of administration and standard pharmaceutical practice.

For example, the components can be administered (e.g. orally or topically) in the form of tablets, capsules, ovules, elixirs, solutions or suspensions, which may contain flavouring or colouring agents, for immediate-, delayed-, modified-, sustained-, pulsed- or controlled-release applications.

If the pharmaceutical is a tablet, then the tablet may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The routes for administration (delivery) include, but are not limited to, one or more of: oral (e.g. as a tablet, capsule, or as an ingestable solution), topical, mucosal (e.g. as a nasal spray or aerosol for inhalation), nasal, parenteral (e.g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

In a preferred aspect, the pharmaceutical composition is delivered topically.

It is to be understood that not all of the components of the pharmaceutical need be administered by the same route. Likewise, if the composition comprises more than one active component, then those components may be administered by different routes.

If a component of the present invention is administered parenterally, then examples of such administration include one or more of: intravenously, intra-arterially, intraperitoneally, intrathecally, intraventricularly, intraurethrally, intrastemally, intracranially, intramuscularly or subcutaneously administering the component; and/or by using infusion techniques.

For parenteral administration, the component is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

As indicated, the component(s) of the present invention can be administered intranasally or by inhalation and is conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A^{™}) or 1,1,1,2,3,3,3-heptafluoropropane (HFA 227EA^{™}), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the agent and a suitable powder base such as lactose or starch.

Alternatively, the component(s) of the present invention can be administered in the form of a suppository or pessary, or it may be applied topically in the form of a gel, hydrogel, lotion, solution, cream, ointment or dusting powder. The component(s) of the present invention may also be dermally or transdermally administered, for example, by the use of a skin patch. They may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the component(s) of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

### Dose Levels

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

Depending upon the need, the agent may be administered at a dose of from 0.01 to 30 mg/kg body weight, such as from 0.1 to 10 mg/kg, more preferably from 0.1 to 1 mg/kg body weight.

### Formulation

The component(s) of the present invention may be formulated into a pharmaceutical composition, such as by mixing with one or more of a suitable carrier, diluent or excipient, by using techniques that are known in the art.

### Pharmaceutically Active Salt

The agent of the present invention may be administered as a pharmaceutically acceptable salt. Typically, a pharmaceutically acceptable salt may be readily prepared by using a desired acid or base, as appropriate. The salt may precipitate from solution and be collected by filtration or may be recovered by evaporation of the solvent.

### Treatment

It is to be appreciated that all references herein to treatment include one or more of curative, palliative and prophylactic treatment. Preferably, the term treatment includes at least curative treatment and/or prophylactic treatment.

The treatment may be of one or more of those disorders mentioned herein, or related complaint.

### Therapy

The agents/modulators identified by the methods of the present invention may be used as therapeutic agents - i.e. in therapy applications.

As with the term "treatment", the term "therapy" includes curative effects, alleviation effects, and prophylactic effects.

The therapy may be on humans or animals.

The therapy can include the treatment of one or more of those disorders mentioned herein, or related complaint.

The invention is now described by way of example in which reference is made to the following figures.

### Brief Description of the Figures

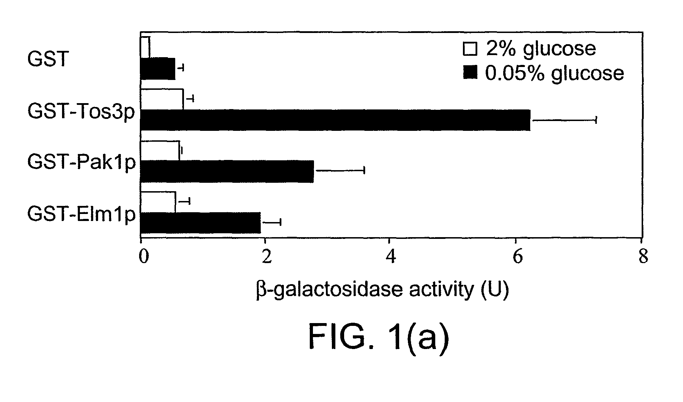

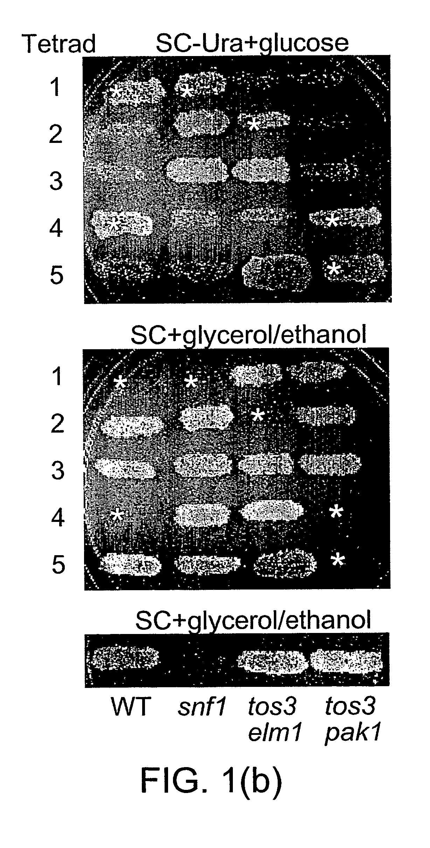

**Fig. 1****.** Overexpression and mutant phenotypes. (A) Overexpression of Tos3p, Pak1p, and Elmlp stimulates Snf1 function in a reporter assay. Transformants of strain TAT7 (*ura3::lexAop-lacZ*) expressed GST or GST-Tos3p, -Paklp, or -Elm1p from a copper-inducible promoter (pRH95, pRH98, pRH94, respectively, purified from a library (Martzen, 1999) and LexA-Snflp (pOV8 (Vincent, 2001). Synthesis of β-galactosidase from the reporter depended on activity of LexA-Snflp (Kuchin, 2000). Transformants (n=3) were grown to mid-log phase in selective synthetic complete (SC) medium + 2% glucose, shifted to medium containing 0.5 mM CuSO₄ and 2% glucose (open bars) or 0.05% glucose (dark bars) for 3 h, and assayed for β-galactosidase activity (Kuchin, 2000). Control transformants expressing LexA with each GST-kinase gave values <0.3 U. (**B**) Triple *tos3Δpak1Δelm1Δ* mutants exhibit growth defects. Double mutants derived from W303, MCY5117 (*MATα tos3Δ::KanMX4 pak1Δ::KanMX4 ura3)* and MCY5122 (*MAT**a** tos3Δ::KanMX4 elm1Δ::URA3 ura3*)*,* were crossed and subjected to tetrad analysis. Segregants were replicated from rich medium to SC-Ura+2% glucose and SC+2% glycerol/3% ethanol. Five tetrads are shown. The same pattern of growth was observed on SC+2% raffinose+antimycin A (1µg/ml). Asterisks indicate triple mutant segregants; genotypes were determined by PCR analysis of genomic DNA. Control strains: wild type (WT); *snf1Δ* mutant; parental double mutants.

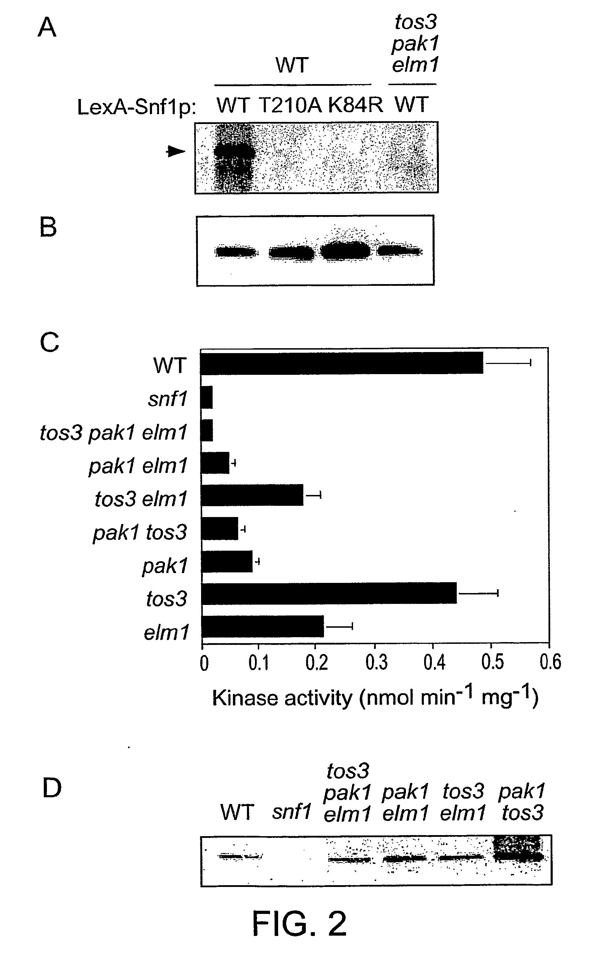

**Fig. 2****.** Assays of Snf1 kinase activity. (**A,B**) Wild-type and triple mutant cells expressing LexA-Snflp or its T210A and K84R mutant derivatives (pRJ55, pRJ217, and pRJ215 (Jiang, 1996) were grown in selective SC+2% glucose. Proteins were immunoprecipitated from extracts (200 µg) with anti-LexA. Immunoprecipitates (**A**) were incubated in kinase buffer containing γ-³²P-ATP and then analyzed by SDS-PAGE and autoradiography and (**B**) were analyzed by immunoblotting with anti-LexA. (**C,D**) Extracts were prepared in duplicate from cells grown in rich medium. Snf1 kinase was partially purified by chromatography on DEAE-Sepharose, and peak fractions were pooled and concentrated. Pooled fractions (**C**) were assayed in triplicate for phosphorylation of the SAMS peptide (HMRSAMSGLHLVKRR) in the presence of γ-³²P-ATP (Woods, 1994; Davies, 1989) and (**D**) were immunoblotted with anti-Snf1. A *snf1-K84R* mutant extract also showed no activity.

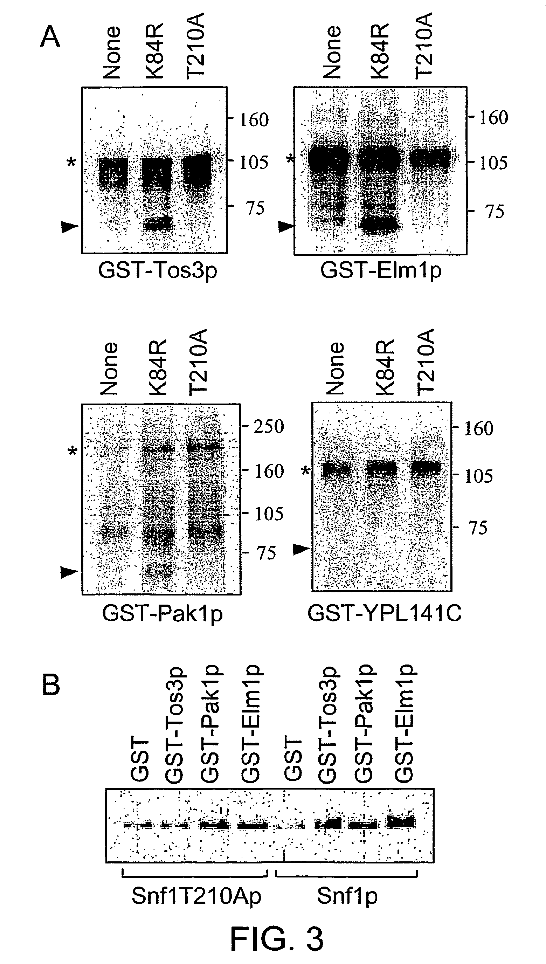

**Fig. 3**. Tos3p and Elmlp phosphorylate Snflp on T210 in vitro. His-tagged Snf1KD-K84R and SnflKD-T210A were bacterially expressed from pRH89 and pRH90, derivatives of pET32c (Novagen), and purified by cobalt affinity chromatography on TALON resin (Clontech). Extracts were prepared from yeast cells expressing GST-Tos3p (Martzen, 1999), GST-Pak1p (Martzen, 1999) and GST-Elm1p (Zhu, 2000), and the GST-kinase was purified on glutathione-Sepharose. The immobilized GST-kinase was incubated with the indicated SnflKD mutant protein (0.2 µg) or no substrate in the presence of γ-³²P-ATP for 20 min at 25°C. Proteins were separated by SDS-PAGE. An autoradiogram is shown. An arbitrary GST-kinase, YPL141C, served as a control. Arrows, SnflKD. Asterisks, autophosphorylated full-length GST-kinase. Molecular size markers (kDa) are indicated.
**Fig. 4**. Tos3p and LKB1 phosphorylate and activate AMPK in vitro. (**A**) Bacterially expressed AMPK (α1β1γ1 (Neumann, 2003)) was incubated with MgATP and GST-kinase bound to glutathione-Sepharose for 30 min at 30°C. Following centrifugation to remove the resin, AMPK activity in the supernatant was measured using the SAMS peptide assay (Davies, 1989). (**B**) A catalytically inactive form of AMPK (2 µg) harboring a D 157A mutation in the α subunit (Stein, 2000) was incubated with GST-Tos3p or GST bound to glutathione-Sepharose beads in the presence of γ-³²P-ATP for 30 min at 30°C. The beads were removed by centrifugation, and proteins in the supernatant were analyzed by SDS-PAGE and autoradiography. Molecular size markers (kDa) are indicated. (**C**) Bacterially expressed AMPK (0.15 µg) was phosphorylated by GST-Tos3p and analysed by immunoblotting with anti-phosphothreonine 172 specific antibody (Cell Signalling Technologies). AMPK was also incubated with GST or partially purified rat liver AMPKK (Hawley, 1996). (**D**) FLAG-tagged LKB1 (wild-type, WT, or a catalytically inactive mutant (D194A); was purified from COS7 cell lysates by binding to FLAG-affinity gel (Sigma). Bacterially expressed AMPK was incubated with the beads and AMPK activity measured as in (A). Phosphorylation of T172 was determined by immunoblotting.

### Examples

### Example 1: Activation of yeast Snf1 and mammalian AMP-activated protein kinase by upstream kinases

### Overview

Snfland AMP-activated protein kinase (AMPK) are the downstream components of protein kinase cascades that play fundamental roles in cellular responses to metabolic stress and appear to be conserved in all eukaryotic cells. In humans, AMPK has been proposed to play a role in metabolic disorders, including diabetes and obesity. The upstream kinase(s) in the cascade have remained elusive, despite extensive efforts. We have identified three kinases, Pak1p, Tos3p and Elm1p, that activate Snf1 kinase in yeast. Triple deletion of the cognate genes causes a mutant phenotype and abolishes Snf1 catalytic activity. All three kinases phosphorylate recombinant Snflp on the activation-loop threonine. Moreover, Tos3p phosphorylates and activates recombinant mammalian AMPK suggesting conservation of function of the upstream kinases. There are no clear mammalian homologues of Pak1p, Tos3p and Elmlp, although their catalytic domains are most closely related to members of the Ca²⁺/calmodulin-dependent protein kinase kinase (CaMKK) family. Previous studies, however, indicate that the major upstream kinase in the AMPK cascade (AMPKK) is not Ca²⁺/calmodulin-dependent.

It is demonstrated herein that the CamKK-related protein kinase, LKB1, phosphorylates and activates AMPK in vitro. Mutations in human LKB cause Peutz-Jeghers syndrome, a hereditary cancer. Withouthese results suggest that the same protein kinase cascade is involved in regulation of metabolism as well as growth and transformation.

The Snfl/AMPK family of kinases is important for metabolic stress responses in eukaryotes. In mammals, AMPK is activated by multiple stresses, many of which lead to an increase in the cellular AMP:ATP ratio, and also by leptin and the hypoglycemic agent metformin. AMPK has a central role in coordinating energy homeostasis and is a major regulator of lipid metabolism, glycogen storage, and glucose transport. AMPK has been implicated in the development and treatment of metabolic disorders, including type 2 diabetes and obesity, and mutations in AMPK cause cardiac abnormalities in humans.

In the yeast *S*. *cerevisiae,* the Snf1 kinase is also required for stress responses, notably the adaptation of cells to carbon stress. Snf1 regulates the transcription of many genes in response to glucose limitation and is required for utilization of alternate carbon sources. Snf1 also has roles in meiosis and sporulation, filamentous invasive growth, and aging.

The Snf1 kinase comprises the catalytic subunit Snf1p (α subunit of AMPK), Snf4p (γ subunit of AMPK), and one of three β subunits (encoded by SIP1, SIP2 and GAL83). Snf4p stimulates kinase activity by counteracting autoinhibition by the Snflp regulatory domain. The β subunit regulates the subcellular localization of the kinase and mediates interactions with downstream targets. The signals that control Snf1 activity in response to glucose levels are not resolved in the prior art, although the AMP:ATP ratio may have some role.

Despite intensive efforts, the identity of the upstream kinases that phosphorylate Snf1 and AMPK have remained elusive. In yeast, genetic approaches have failed to yield mutations in the cognate gene, suggesting that multiple kinases activate Snf1.

Snf1 is activated by phosphorylation and the activation-loop threonine residue, T210, is critical for kinase activity.

### Example 2: Tos3p, Elm1p and Pak1p activate Snf1

Mass spectrometric analysis of yeast protein complexes indicated that Tos3p (YGL179C) copurifies with Snf4p, and Pak1p (unrelated to mammalian p21-activated kinase) copurifies with Snflp and with Snf4p. Tos3p and Pak1p are closely related, but their functions are unknown except that Pak1 suppresses DNA polymerase mutations.

We demonstrate that Tos3p, Elmlp and Pak1p activate Snf1.

We demonstrate activation of yeast AMPK (Snf1) by upstream kinases (Elm1p, Tos3p and Pak1p).

Exemplary assay of yeast AMPK (Snf1) is disclosed.

To confirm that Tos3p interacts with Snflp, we expressed a glutathione-*S*-transferase (GST) fusion to Tos3p in yeast and demonstrated that LexA-tagged Snflp copurified on glutathione-Sepharose.

We introduced *tos3Δ* and *pak1Δ* mutations into yeast cells and tested for phenotypes characteristic of a *snf1Δ* mutant. The single and double mutants showed no defect in growth on raffinose or glycerol/ethanol (see Fig. 1B).

We then sought other genetic evidence that Tos3p and Pak1p are functionally related to the Snf1 kinase. We reasoned that if they activate Snf1, their overexpression might compensate for the absence of the stimulatory subunit Snf4p. Overexpression of GST-Tos3p or GST-Pak1p partially suppressed *snf4Δ* mutant defects in *SUC2* (invertase) gene expression, and GST-Tos3p restored growth on raffinose in a *snf4Δ* mutant, but did not bypass the requirement for Snf1.

We also used an assay in which transcription of a *lexAop-lacZ* reporter depends on the catalytic activity of LexA-Snflp bound to the promoter (Kuchin, 2000-incorporated herein by reference).

Overexpression of GST-Tos3p or GST-Pak1p stimulated β-galactosidase synthesis in response to glucose limitation, implying a positive effect on LexA-Snflp catalytic activity (Fig. 1A).

The lack of phenotype in the double mutant suggested that another kinase provides redundant function. The kinase most closely related to Pak1p and Tos3p is Elm1p, which was identified by a mutation that causes elongated cell morphology and affects pseudohyphal development. Elmlp has roles in the control of bud growth and cytokinesis.

We introduced *elm1Δ* into the above mutant strains by gene disruption. The *tos3Δ elm1Δ* and *pak1Δ elm1Δ* strains grew on raffinose and glycerol/ethanol, but the triple *tos3Δ pak1Δelm1Δ* mutant did not. To confirm this result, we crossed *tos3Δpak1Δ* and *tos3Δ elm1Δ* strains and carried out tetrad analysis. Fourteen triple mutant segregants from thirteen tetrads were defective in growth on glycerol/ethanol and raffinose (Fig. 1B). Assays of invertase activity showed that *SUC2* expression was abolished (95 U in derepressed wild-type cells and <1 U in *snf1Δ* and triple mutant cells). The triple mutant also manifested a defect in glycogen accumulation, another *snf1Δ* mutant phenotype. These genetic findings support the view that a function provided redundantly by Tos3p, Pak1p and Elm1p is required for Snf1 kinase function in vivo.

### Example 3: Activation of AMPK (Snf1) by upstream kinase

To determine whether these three kinases activate AMPK (Snf1), we used an in vitro kinase assay. Protein extracts were prepared from wild-type and triple mutant cells expressing LexA-Snflp. LexA-Snflp was immunoprecipitated with anti-LexA and incubated in the presence of γ-³²P-ATP. When immunoprecipitated from the wild-type extract, LexA-Snflp was phosphorylated in vitro, and controls with catalytically inactive LexA-Snf1K84R and LexA-Snf1T210A (carrying substitutions of the ATP-binding site lysine and the activation-loop threonine, respectively) confirmed that Snf1 kinase activity was responsible. In contrast, when LexA-Snflp was precipitated from the triple mutant, no phosphorylation was detected (Fig. 2A), despite equivalent protein levels (Fig. 2B).

### Example 4: Assay of AMPK (Snf1) activity

We also assayed AMPK (Snfl) catalytic activity in vitro by phosphorylation of the SAMS synthetic peptide substrate [Woods, 1994 #507; Davies, 1989 #526]. Snf1 was partially purified from cell extracts, under conditions that activate the kinase [Wilson, 1996 #463; Woods, 1994 #507], and was incubated with SAMS peptide in the presence of γ-³²P-ATP. The peptide was phosphorylated in assays of wild-type but not *snf1Δ* extracts. No activity was detected in the *tos3Δ pak1Δ elm1Δ* mutant (Fig. 2C); immunoblot analysis confirmed the presence of Snflp (Fig. 2D). These results indicate that Snf1 remains inactive in the absence of upstream kinase such as Tos3p, Pak1p and Elm1p.

### Example 5: Activation of AMPK (Snf1) by upstream kinase

To demonstrate that these three kinases directly phosphorylate Snflp, we used as a substrate an inactive form of the isolated Snf1 catalytic domain, designated SnflKD-K84R (Fig. 3). GST-Tos3p, GST-Pak1p, and GST-Elm1p were purified from yeast and incubated with bacterially expressed Snf1KD-K84R in the presence of γ-³²P-ATP. GST-Tos3p and GST-Elmlp and GST-Pak1p phosphorylated Snf1KD-K84R. GST-Pak1p phosphorylated the substrate weakly, perhaps because only minimal amounts of full-length fusion protein were recovered. Incubation with an unrelated GST-kinase, YPL141C, confirmed that Snf1KD-K84R was not autophosphorylated.

To demonstrate that Tos3p and Elm1p phosphorylate the activation-loop threonine, we used a mutant substrate lacking T210, Snf1KD-T210A. No phosphorylation was detected (Fig. 3).

### Example 6: Activation of AMPK (mammalian) by upstream kinase

We next demonstrate the ability of the purified yeast GST-kinases to phosphorylate and activate mammalian AMPK. Recombinant AMPK (α1β1γ1) expressed in bacteria (Neumann, 2003) was incubated with each of the kinases and MgATP, and AMPK activity was determined using the SAMS peptide assay.

Incubation with GST-Tos3p led to a marked increase in AMPK activity, whereas GST-Elmlp and GST-Pak1p caused only a small increase (Fig. 4A).

GST-Tos3p phosphorylated the α subunit of a catalytically inactive form of AMPK, but not the β or γ subunit (Fig. 4B). Immunoblot analysis showed that GST-Tos3p phosphorylates AMPK on T172 (Fig. 4C). These findings suggest functional conservation of the upstream kinases between yeast and mammals.

### Example 7: Activation of AMPK (mammalian) by upstream kinase (mammalian)

Tos3p, Pak1p and Elmlp are most closely related to mammalian Ca²⁺/calmodulin-dependent protein kinase kinase (CaMKK), although they may have diverged from the yeast group of CaMKs. Mammalian CaMKK weakly phosphorylates and activates AMPK in vitro, but the major AMPK kinase (AMPKK) is distinct from CaMKK.

As disclosed herein, these findings confirm that AMPKK corresponds to another CaMK-related kinase(s). At least one major AMPKK is the LKB1 (STK11) tumor suppressor kinase, which is mutated in Peutz-Jeghers syndrome.

FLAG-tagged LKB1 (full length wild-type, WT, or a catalytically inactive mutant (D 194A); (mouse cDNA in pCDNA3 vector flag tagged) was purified from COS7 cell lysates by binding to FLAG-affinity gel (Sigma). Bacterially expressed AMPK was incubated with the beads and AMPK activity measured.

Bacterially expressed AMPK (α1β1γ1 (Neumann, 2003)) was incubated with MgATP and FLAG-LKB1 kinase bound to FLAG-affinity gel for 30 min at 30°C In another aspect, the invention relates to shaking incubator.

Specifically, the buffer conditions of AMPK are adjusted to final concentration 100uM ATP, 5mM MgC12, 50mM HEPES pH7.5, 10% glycerol, 1mM EDTA, 1mM DTT, at the point that FLAG-LKB1 gel is added.

Following centrifugation to remove the resin, AMPK activity in the supernatant was measured using the SAMS peptide assay (Davies, 1989).

LKB1, expressed in mammalian cells, phosphorylated T172 and activated recombinant AMPK in vitro (Fig. 4D).

Phosphorylation of T172 was determined by immunoblotting (FIG 4E).

Thus it is demonstrated that LKB1 phosphorylates and activates AMPK.

### Example 8: Expression of LKB1 in eukaryotic cells

A polymerase chain reaction-based strategy was used to prepare an N-terminal FLAG epitope-tagged cDNA construct encoding mouse LKB1 using, as a template, an expressed sequence tag encoding full-length mouse LKB1 (NCBI accession number AA542163, IMAGE number 550355) obtained from the IMAGE consortium. The construct was obtained using the 59-primer:
atgcatactagtgccaccatggactactacaaggacgacgatgacaaggacgtggcggaccccgagccgttggg
and the 39-primer: gacagaactagttcactgctgcttgcaggccgaga.

To prepare the catalytically inactive mutant of LKB1 termed LKB1 (KD), where KD is kinase-dead, Asp194 in subdomain VII of the kinase domain is mutated to Ala.

Naturally, any homologue such as a mammalian homologue of LKB1 could be used instead of the mouse, such as human LKB1. Equally, the LKB1 could be expressed with or without the flag epitope tag, or could be expressed with an alternative tag such as myc, HA, glutathione-*S*-transferase or other tag.

### Expression of LKB1 in mammalian cells

The resulting polymerase chain reaction fragment was subcloned as an *Eco*RI*-Eco*RI fragment into the pCDNA3 vector (Invitrogen) to encode expression of FLAG-LKB1 in mammalian cells.

### Expression of LKB1 in yeast

Mouse LKB1 (containing a myc epitope tag) was subcloned into the yeast expression vector pYX212 (R and D Systems) and expressed in yeast.

### Example 9: Phosphorylation and assay of AMPK

Bacterially expressed AMPK (α1β1γ1) was purified by chromatography using Ni-NTA agarose (Qiagen) as previously described (the purification is as in Neumann et al., 2003 Protein Expression and Purification, incorporated herein by reference). AMPK was incubated with 100 µM ATP, 5 mM MgCl₂, 200 µM AMP and 1 mM DTT in 50 mM Hepes, pH 7.4 in the presence or absence of upstream kinase for 30 min at 30°C. Following brief centrifugation, the supernatant containing AMPK was removed and activity measured using the SAMS peptide assay (Davies et al., 1989). Phosphorylation of T172 was determined by Western blotting using an antibody that specifically recognizes phospho-threonine 172 within the α subunit of AMPK (Cell Signaling Technologies).

³²P-phosphate labelling of AMPK was analysed by incubating a catalytically inactive form of AMPK (harbouring the mutation D157A in the α subunit) in the presence of γ-³²P-ATP in the presence or absence of upstream kinase. In this example the upstream kinase is LKB1.

### Expression of LKB1

Plasmid DNA encoding either FLAG-tagged wild-type LKB1 (mouse) or FLAG-tagged catalytically inactive LKB1 (harbouring a D194A mutation) was transfected into COS7 cells using lipofectamine reagent. Cells were harvested 48 h post-transfection and LKB1 protein immunoprecipitated with EZview Red M2 FLAG affinity gel (Sigma) and used as required such as in incubations with AMPK.

AMPK in the supernatant was resolved by SDS-PAGE followed by autoradiography.

Various modifications and variations of the described methods and systems of the present invention will be apparent to those skilled in the art without departing from the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry, molecular biology/genetics and biotechnology or related fields are intended to be within the scope of the following claims.

## Claims

1. An *in vitro* method for assaying LKB1 activity comprising
(i) providing a sample comprising LKB1,
(ii) contacting said sample with a substrate kinase under conditions permissive of phosphorylation, wherein said substrate kinase is or is derived from AMPK, and
(iii) monitoring incorporation of phosphate into said substrate kinase
wherein the incorporation of phosphate into said substrate kinase indicates LKB1 activity.

2. A method according to claim 1 wherein the AMPK comprises heterotrimeric AMPK.

3. A method according to claim 1 wherein the AMPK comprises mouse or human AMPK.

4. A method according to any of claims 1 to 3 wherein incorporation of phosphate into AMPK is monitored by assaying AMPK activity.

5. A method according to claim 4 wherein AMPK activity is assayed via phosphorylation of SAMS peptide.

6. A method according to any of claims 1 to 3 wherein incorporation of phosphate into AMPK is monitored by assaying phosphorylation of T-172 of AMPK, or the equivalent thereof.

7. A method according to any of claims 1 to 3 further comprising quantifying the amount of phosphate incorporated per mol of AMPK, wherein said phosphate level indicates the level of LKB1 present in said sample.

8. *In vitro* use of recombinant or isolated LKB1 as an AMPKK.

9. *In vitro* use according to claim 8 in the phosphorylation of AMPK.

10. *In vitro* use according to claim 8 or claim 9 in the activation of AMPK.

11. Use of LKB1 in the manufacture of a medicament for the prevention or treatment of Peutz-Jeghers Syndrome (PJS).

12. *In vitro* use of HSP90, Cdc37, or STRAD protein in the modulation of AMPK activity.

13. An *in vitro* method of modulating AMPK comprising modulating LKB1.

14. A method according to claim 13 wherein said method comprises increasing the phosphorylation of AMPK in a system by increasing the activity of LKB1 in said system.

15. A method according to claim 13 wherein said method comprises activating AMPK in a system by activating LKB1 in said system.

16. A method according to claim 13 wherein said method comprises phosphorylating AMPK by contacting AMPK with recombinant or isolated LKB1.

17. A method according to claim 13 wherein said method comprises activating AMPK by contacting said AMPK with recombinant or isolated LKB1.

18. An *in vitro* method for identification of modulator(s) of LKB1 comprising
(i) providing a first and a second sample of LKB 1,
(ii) contacting said first sample with a candidate modulator of LKB 1,
(iii) contacting said first and second samples with a substrate kinase under conditions permissive of phosphorylation, wherein said substrate kinase is or is derived from AMPK,
(iv) monitoring incorporation of phosphate into said substrate, and comparing the incorporation of phosphate into said substrate in said first and second samples
wherein if the incorporation of phosphate into the substrate differs between said first and second samples, the candidate modulator is identified as a modulator of LKB1 activity.

19. A recombinant yeast cell comprising gene disruptions in at least two of Pak1, Tos3 and Elm1.

20. A recombinant yeast cell according to claim 19 comprising gene disruptions in Pak1, Tos3 and Elm1.

21. A recombinant yeast cell according to claim 19 or 20 further comprising a gene disruption in Snf1.

22. A recombinant yeast cell according to any of claims 19 to 21 which expresses mammalian AMPK.

23. A recombinant yeast cell according to claim 22 wherein the mammalian AMPK comprises alpha, beta and gamma subunits.

## Patentansprüche

1. In-Vitro-Verfahren zur Testung der LKB1-Aktivität, umfassend
(i) das Bereitstellen einer Probe, die LKB1 umfasst,
(ii) das In-Kontakt-Bringen der Probe mit einer Substratkinase unter Bedingungen, die eine Phosphorylierung ermöglichen, wobei die Substratkinase AMPK ist oder von dieser abgeleitet ist, und
(iii) das Aufzeichnen der Eingliederung von Phosphat in die Substratkinase, wobei die Eingliederung von Phosphat in die Substratkinase eine LKB1-Aktivität anzeigt.

2. Verfahren nach Anspruch 1, wobei die AMPK heterotrimere AMPK umfasst.

3. Verfahren nach Anspruch 1, wobei die AMPK Maus- oder Human-AMPK umfasst.

4. Verfahren nach einem der Ansprüche 1 bis 3, wobei die Eingliederung von Phosphat in AMPK durch Testen der AMPK-Aktivität aufgezeichnet wird.

5. Verfahren nach Anspruch 4, wobei die AMPK-Aktivität durch die Phosphorylierung von SAMS-Peptid getestet wird.

6. Verfahren nach einem der Ansprüche 1 bis 3, wobei die Eingliederung von Phosphat in AMPK durch Testen der Phosphorylierung von T-172 von AMPK oder des Äquivalents davon aufgezeichnet wird.

7. Verfahren nach einem der Ansprüche 1 bis 3, des Weiteren umfassend das Quantifizieren der Menge an Phosphat, die pro Mol AMPK eingegliedert ist, wobei der Phosphatwert den Wert von LKB1 angibt, der in der Probe vorhanden ist.

8. In-Vitro-Verwendung von rekombinantem oder isoliertem LKB1 als eine AMPKK.

9. In-Vitro-Verwendung nach Anspruch 8 in der Phosphorylierung von AMPK.

10. In-Vitro-Verwendung nach Anspruch 8 oder Anspruch 9 in der Aktivierung von AMPK.

11. Verwendung von LKB1 in der Herstellung eines Medikaments zur Prävention oder Behandlung des Peutz-Jeghers-Syndroms (PJS).

12. In-Vitro-Verwendung des HSP90-, Cdc37- oder STRAD-Proteins in der Modulierung der AMPK-Aktivität.

13. In-Vitro-Verfahren zur Modulierung von AMPK, umfassend die Modulierung von LKB1.

14. Verfahren nach Anspruch 13, wobei das Verfahren das Erhöhen der Phosphorylierung von AMPK in einem System durch Erhöhen der Aktivität in LKB1 in diesem System umfasst.

15. Verfahren nach Anspruch 13, wobei das Verfahren das Aktivieren von AMPK in einem System durch Aktivieren von LKB1 in diesem System umfasst.

16. Verfahren nach Anspruch 13, wobei das Verfahren das Phosphorylieren von AMPK durch In-Kontakt-Bringen von AMPK mit rekombinantem oder isoliertem LBK1 umfasst.

17. Verfahren nach Anspruch 13, wobei das Verfahren das Aktivieren von AMPK durch In-Kontakt-Bringen von AMPK mit rekombinantem oder isoliertem LBK1 umfasst.

18. In-Vitro-Verfahren zur Identifizierung eines oder mehrerer Modulatoren von LBK1, umfassend:
(i) das Bereitstellen einer ersten und einer zweiten Probe von LKB1,
(ii) das In-Kontakt-Bringen der ersten Probe mit einem Kandidat-Modulator von LKB1,
(iii) das In-Kontakt-Bringen der ersten und der zweiten Probe mit einer Substratkinase unter Bedingungen, die eine Phosphorylierung ermöglichen, wobei die Substratkinase AMPK ist oder von dieser abgeleitet ist, und
(iv) das Aufzeichnen der Eingliederung von Phosphat in das Substrat, und Vergleichen der Eingliederung von Phosphat in das Substrat in der ersten und zweiten Probe,
wobei, wenn sich die Eingliederung von Phosphat in das Substrat zwischen der ersten und zweiten Probe unterscheidet, der Kandidat-Modulator als Modulator der LKB1-Aktivität identifiziert ist.

19. Rekombinante Hefezelle, umfassend Gendisruptionen in mindestens zwei von Pak1, Tos3 und Elm1.

20. Rekombinante Hefezelle nach Anspruch 19, umfassend Gendisruptionen in Pak1, Tos3 und Elm1.

21. Rekombinante Hefezelle nach Anspruch 19 oder 20, des Weiteren umfassend Gendisruptionen in Snf1.

22. Rekombinante Hefezelle nach einem der Ansprüche 19 bis 21, die Säugetier-AMPK exprimiert.

23. Rekombinante Hefezelle nach Anspruch 22, wobei die Säugetier-AMPK Alpha-, Beta- und Gamma-Subeinheiten umfasst.

## Revendications

1. Procédé *in vitro* de dosage de l'activité de la LKB1 comprenant
(i) la fourniture d'un échantillon comprenant la LKB1,
(ii) la mise en contact dudit échantillon avec une kinase de substrat dans des conditions qui permettent la phosphorylation, où ladite kinase de substrat est une AMPK ou est obtenue à partir de l'AMPK, et
(iii) la surveillance de l'incorporation de phosphate dans ladite kinase de substrat, où l'incorporation de phosphate dans ladite kinase de substrat indique l'activité de la LKB1.

2. Procédé selon la revendication 1, dans lequel l'AMPK comprend une AMPK hétérotrimérique.

3. Procédé selon la revendication 1, dans lequel l'AMPK comprend une AMPK de souris ou d'être humain.

4. Procédé selon l'une quelconque des revendications 1 à 3, dans lequel l'incorporation de phosphate dans l'AMPK est surveillée par le dosage de l'activité de l'AMPK.

5. Procédé selon la revendication 4, dans lequel l'activité de l'AMPK est dosée par l'intermédiaire d'une phosphorylation de peptide SAMS.

6. Procédé selon l'une quelconque des revendications 1 à 3, dans lequel l'incorporation de phosphate dans l'AMPK est surveillée en dosant la phosphorylation de T-172 de l'AMPK, ou de son équivalent.

7. Procédé selon l'une quelconque des revendications 1 à 3, comprenant en outre la quantification de la quantité de phosphate incorporée par mole d'AMPK, où ledit niveau de phosphate indique le niveau de la LKB1 présente dans ledit échantillon.

8. Utilisation *in vitro* de la LKB1 recombinante ou isolée en tant que AMPKK.

9. Utilisation *in vitro* selon la revendication 8, dans la phosphorylation d'AMPK.

10. Utilisation *in vitro* selon la revendication 8 ou la revendication 9, dans l'activation d'AMPK.

11. Utilisation de la LKB1 dans la fabrication d'un médicament destiné à la prévention ou au traitement du syndrome de Peutz-Jeghers (PJS).

12. Utilisation *in vitro* de la protéine HSP90, CdC37 ou STRAD, dans la modulation de l'activité de l'AMPK.

13. Procédé *in vitro* de modulation de l'AMPK comprenant la modulation de la LKB1.

14. Procédé selon la revendication 13 où ledit procédé comprend une augmentation de la phosphorylation de l'AMPK dans un système en augmentant l'activité de la LKB1 dans ledit système.

15. Procédé selon la revendication 13, où ledit procédé comprend une activation de l'AMPK dans un système en activant la LKB1 dans ledit système.

16. Procédé selon la revendication 13, où avec ledit procédé comprend une phosphorylation de l'AMPK en mettant en contact l'AMPK avec une LKB1 recombinante ou isolée.

17. Procédé selon la revendication 13, où ledit procédé comprend une activation de l'AMPK en mettant en contact ladite AMPK avec une LKB1 recombinante ou isolée.

18. Procédé *in vitro* d'identification de modulateur(s) de la LKB1 comprenant
(i) la fourniture d'un premier et d'un deuxième échantillons de LKB1,
(ii) la mise en contact dudit premier échantillon avec un modulateur candidat de LKB1,
(iii) la mise en contact desdits premier et deuxième échantillons avec une kinase de substrat dans des conditions qui permettent la phosphorylation, où ladite kinase de substrat est l'AMPK ou est obtenue à partir de l'AMPK,
(iv) la surveillance de l'incorporation de phosphate dans ledit substrat, et la comparaison de l'incorporation de phosphate dans ledit substrat dans lesdits premier et deuxième échantillons
où, si l'incorporation de phosphate dans le substrat diffère entre lesdits premier et deuxième échantillons, le modulateur candidat est identifié comme étant un modulateur de l'activité de la LKB 1.

19. Cellule de levure recombinante comprenant des perturbations géniques dans au moins deux parmi la Pak1, la Tos3 et l'Elm1.

20. Cellule de levure recombinante selon la revendication 19, comprenant des perturbations géniques dans la Pak1, la Tos3 et l'Elm1.

21. Cellule de levure recombinante selon la revendication 19 ou 20, comprenant en outre une perturbation génique dans la Snf1.

22. Cellule de levure recombinante selon l'une quelconque des revendications 19 à 21, laquelle exprime une AMPK de mammifère.

23. Cellule de levure recombinante selon la revendication 22, dans laquelle l'AMPK de mammifère comprend des sous-unités alpha, béta et gamma.
